# EUROPÄISCHE PATENTANMELDUNG

(11) **EP 1 527 796 A2**
(43) Veröffentlichungstag der Anmeldung: **04.05.2005**
(21) Anmeldenummer: 04021783.8
(22) Anmeldetag: 14.09.2004
(51) Int. Cl.: A61M 15/00

(54) **Inhalator**

(30) Priorität: 29.10.2003 DE 10350555
(71) Anmelder: Hugo Kern und Liebers GmbH & Co. Platinen- und Federnfabrik, 78713 Schramberg (DE)
(72) Erfinder: Frietsch, Klaus, 78713 Schramberg (DE); Roming, Paul, 78713 Schramberg (DE); Hettich, Richard, 78713 Schramberg (DE); Pabst, Joachim, Dr., 64354 Reinheim (DE)
(74) Vertreter: Mussgnug, Bernd, Dr.rer.nat.

(57) **Zusammenfassung**

Ein Inhalator weist eine antreibbare Frässcheibe (14) auf, gegen die eine feste Wirkstofftablette (18) zur Anlage gebracht wird. Die Frässcheibe (14) ist über einen Federantrieb oder Elektromotor antreibbar, um Wirkstoffpartikel von der Wirkstofftablette (18) abzutragen. Bei der Benutzung des Inhalators wird beim Einatmen Luft über Luftkanäle (24, 26, 28) und ein Mundstück (12) angesaugt, die die abgetragenen Wirkstoffpartikel mitnimmt. Um den Drehantrieb der Frässcheibe (14) auszulösen, ist eine Luftklappe (60) in den Strömungsweg der angesaugten Luft eingesetzt. Die Luftklappe (60) wird beim Einatmen durch die angesaugte Luftströmung verschwenkt und löst den Drehantrieb aus.

## Beschreibung

Die Erfindung betrifft einen Inhalator gemäß dem Oberbegriff des Anspruchs 1.

Inhalatoren dieser Gattung dienen dazu, den Atemwegen eines Benutzers Wirkstoffe, insbesondere therapeutische Wirkstoffe, zuzuführen. Um eine genau dosierte Menge des Wirkstoffes zuzuführen, wird der Wirkstoff zusammen mit einem Trägermaterial in Form einer Tablette gepresst und eine gewisse Menge der Tablette bei der Benutzung durch eine Abtragseinrichtung in Form von Pulver oder kleinen Partikeln abgetragen, die von dem Benutzer eingeatmet werden.

Aus der DE 295 01 527 U1 ist ein solcher Inhalator bekannt, bei welchem die Wirkstoffpartikel von der Tablette mittels einer Frässcheibe abgetragen werden, die durch eine Feder, insbesondere eine Schenkelfeder drehangetrieben wird. Die Feder wird über einen Freilauf bis zu einem Anschlag gespannt und dreht nach Auslösung die Frässcheibe um einen vorgegebenen Drehweg, durch welchen die abgetragene Menge der Wirkstoffpartikel definiert ist. Die Drehgeschwindigkeit der Frässcheibe wird durch einen Hemmregler bestimmt, so dass ein gleichmäßiger Abtrag der Wirkstoffpartikel gewährleistet ist.

Bei dieser bekannten Vorrichtung wird die Auslösung des gespannten Drehantriebs durch einen Auslöseknopf bewirkt, der von dem Benutzer gedrückt wird. Um die abgetragenen Wirkstoffpartikel zuverlässig und vollständig in die zu therapierenden Atemwegsbereiche zu bringen, ist es notwendig, dass der Benutzer synchron mit der Betätigung des Abtrags einatmet. Erfolgt die Betätigung des Inhalators und damit der Wirkstoffabtrag zeitlich gegenüber dem Einatmen versetzt, so werden die Partikel gar nicht eingeatmet oder können sich die Wirkstoffpartikel im Mund und Rachenraum des Benutzers niederschlagen und gelangen nicht an die zu therapierenden Atemwegsbereiche. Bei dem bekannten Inhalator hängt die Effektivität der Wirkstoffzuführung daher davon ab, dass der Benutzer den Auslöseknopf exakt synchron mit seinem Einatmen betätigt.

Der Erfindung liegt die Aufgabe zu Gründe, einen Inhalator der eingangs genannten Gattung zur Verfügung zu stellen, bei welchem eine exakte Synchronisation zwischen dem Einatmen des Benutzers und der Betätigung der Abtrageinrichtung gewährleistet ist.

Diese Aufgabe wird erfindungsgemäß gelöst'durch einen Inhalator mit den Merkmalen des Anspruchs 1.

Vorteilhafte Ausführungen der Erfindung sind in den Unteransprüchen angegeben.

Der wesentliche Gedanke der Erfindung besteht darin, den Drehantrieb für die Abtrageinrichtung durch den Atemzug des Benutzers auszulösen. Das Einatmen und das Auslösen des Drehantriebs sind dadurch nicht mehr zwei Vorgänge, die der Benutzer willentlich synchronisieren muss. Der Benutzer muss den Inhalator nur an den Mund ansetzen und einatmen, wodurch der Drehantrieb ausgelöst wird und die Wirkstoffpartikel automatisch exakt im Moment des Einatmens abgetragen werden und dadurch vollständig in die Atemwege des Benutzers gelangen. Darüber hinaus wird selbstverständlich die Handhabung des Inhalators vereinfacht, da bei der Benutzung keine Betätigung mehr notwendig ist. Dies ist von Vorteil, wenn die Verwendung des Inhalators z. B. bei Asthmaanfällen mit Angst- oder Panikzuständen verbunden ist. Ebenso ist dies von Vorteil, wenn der Inhalator von kleinen Kindern benutzt werden muss, z. B. von Mukoviszidose-Patienten.

In einer vorteilhaften Ausführung wird die Auslösung des Drehantriebs dadurch bewirkt, dass die von dem Benutzer eingeatmete Luft durch einen Luftkanal des Inhalators angesaugt wird, in welchem sich eine Luftklappe befindet, die durch den Luftstrom verschwenkt wird. Die Schwenkbewegung der Luftklappe löst den Drehantrieb aus.

Es ist ohne Weiteres ersichtlich, dass die atemzuggesteuerte Auslösung bei jeder Art von Drehantrieb verwendet werden kann. Beispielsweise kann der Drehantrieb als elektromotorischer batteriegespeister Antrieb ausgebildet sein. In diesem Falle wird durch die atemzuggesteuerte Auslösung ein elektrischer Kontakt betätigt, der den Drehantrieb in Gang setzt. In einer bevorzugten Ausführung wird als Drehantrieb ein Federantrieb verwendet, da dieser zum einen konstruktiv einfach und preisgünstig ist und zum anderen nicht vom Ladezustand einer Batterie abhängig ist.

Bei der Verwendung eines Federantriebs wird die Antriebsfeder gespannt und der Ablauf des Federantriebs durch ein Sperrglied gesperrt. Das Sperrglied wird durch die atemzuggesteuerte Auslösung, z. B. durch die Bewegung der Luftklappe aus seiner Sperrstellung bewegt und gibt den Federantrieb frei, so dass die Abtrageinrichtung angetrieben werden kann.

Für die mechanische Auslösung eines Federantriebs ist es wichtig, dass die Auslösebewegung mit einem geringen Widerstand erfolgen kann, damit eine zuverlässige Auslösung durch den Atemzug des Benutzers gewährleistet ist. Hierbei hat sich eine schwenkbar gelagerte großflächige Luftklappe als vorteilhaft erwiesen. Eine großflächige Luftklappe hat den Vorteil, dass bereits ein geringer durch den Atemzug bewirkter Druckunterschied an den beiden Flächen der Luftklappe zu einer Schwenkund Öffnungsbewegung führt. Die schwenkbare Luftklappe ist dabei so ausgebildet, dass ihr Masseschwerpunkt möglichst nahe an der Schwenkachse liegt und vorzugsweise mit der Schwenkachse zusammenfällt. Die Schließkraft der Luftklappe wird dabei durch eine Schließfeder erzeugt. Diese Ausbildung hat den Vorteil, dass die Schließkraft der Luftklappe durch die Schließfeder genau definiert werden kann. Die Schließkraft ist dabei von der räumlichen Orientierung des Inhalators unabhängig. Der Inhalator kann in gleicher Weise von einer liegenden oder aufrecht sitzenden oder stehenden Person benutzt werden. Es ist außerdem sichergestellt, dass der Drehantrieb nicht unbeabsichtigt durch Erschütterungen ausgelöst wird, wenn der Inhalator in der Tasche mitgeführt wird.

In einer bevorzugten Ausführung wird ein Federantrieb mit einem Hemmregler verwendet, der einen gleichmäßigen Ablauf des Drehantriebs mit vorgegebener Drehgeschwindigkeit gewährleistet. In dieser Ausführung kann die den Drehantrieb auslösende Luftklappe so gelagert sein, dass sie in der Schließstellung in den Hemmregler, vorzugsweise in dessen Anker eingreift, so dass sie den Hemmregler und damit den Drehantrieb blockiert. Bei dieser Ausführung muss zum Verschwenken der Luftklappe und zur Auslösung des Drehantriebs nur eine geringe Reibung zwischen der Luftklappe und dem Hemmregler überwunden werden.

Bei der Verwendung eines Federantriebs wird der Antrieb zweckmäßig so ausgelegt, dass der Antriebsweg in dem Bereich der Federcharakteristik liegt, in welchem die Federkraft im Wesentlichen konstant und wegunabhängig ist. Dadurch wird eine gleichmäßige Drehgeschwindigkeit und ein gleichmäßiges Drehmoment der Abtrageinrichtung erreicht, so dass der Abtrag der Wirkstoffpartikel über den gesamten Antriebsbereich konstant und gut definiert ist. Ein Anschlag begrenzt das Spannen der Feder in einem Bereich mit im Wesentlichen waagerecht verlaufender Federcharakteristik. Dabei kann eine mechanische elastische Verformung des Anschlags eine starke Rückstellkraft verursachen, die sich der Federkraft zu Beginn des Antriebs überlagert. Um diesen unerwünschten Effekt zu beseitigen, kann in einer Ausführung das Spannen der Antriebsfeder über einen Schleppanschlag mit Pufferfeder erfolgen, der am Ende des Spannweges eine geringe Entspannung der Antriebsfeder bewirkt, wodurch elastische Verformungsspannungen der mechanischen Teile entlastet werden.

Im Folgenden wird die Erfindung anhand eines in der Zeichnung dargestellten Ausführungsbeispiels näher erläutert. Es zeigen:
- Figur 1: einen Axialschnitt durch den Inhalator,
- Figur 2: eine perspektivische Darstellung des Inhalators ohne Gehäuse mit sperrender Luftklappe,
- Figur 3: eine Seitenansicht des Inhalators ohne Gehäuse mit sperrender Luftklappe,
- Figur 4: eine Einzeldarstellung der sperrender Luftklappe und des Ankerringes,
- Figur 5: eine perspektivische Darstellung des Inhalators ohne Gehäuse mit auslösender Luftklappe,
- Figur 6: eine Figur 3 entsprechende Darstellung mit auslösender Luftklappe,
- Figur 7: eine Figur 4 entsprechende Einzeldarstellung mit auslösender Luftklappe,
- Figur 8: eine axiale Stirnansicht der Spanneinrichtung von Innen,
- Figur 9: einen Axialschnitt der Spanneinrichtung der Figur 8,
- Figur 10: einen Querschnitt der Spanneinrichtung gemäß der Schnittlinie A-A in Figur 11,
- Figur 11: einen gegenüber Figur 9 um 90°C gedrehten Axialschnitt der Spanneinrichtung,
- Figur 12: eine axiale Stirnansicht der Spanneinrichtung ohne Schleppring,
- Figur 13: eine Einzeldarstellung der Pufferfederscheibe und
- Figur 14: die Pufferfederscheibe und den Schleppring in perspektivischer Darstellung.

Der Inhalator weist ein hohlzylindrisches Gehäuse 10 auf, auf dessen vorderes Ende ein Mundstück 12 aufschnappbar ist, welches der Benutzer in den Mund einführt. In dem Gehäuse 10 ist eine Abtrageinrichtung in Form einer Frässcheibe 14 in später beschriebener Weise drehbar angeordnet. In dem Mundstück 12 ist koaxial beweglich eine Trägerhülse 16 angeordnet, die eine Wirkstofftablette 18 trägt. Die Wirkstofftablette 18 hat die Form einer Kreisringscheibe und besteht aus einem gepressten pulverförmigen Wirkstoff. Eine Schraubendruckfeder 20 stützt sich einerends an dem Mundstück 12 und anderenends an der Trägerhülse 16 ab. Durch die Schraubendruckfeder 20 wird die in der Trägerhülse 16 befestigte Wirkstofftablette 18 gegen die Stirnfläche der Frässcheibe 14 gepresst, so dass die Frässcheibe 14 Wirkstoffpartikel von der Wirkstofftablette 18 abträgt, wenn die Frässcheibe 14 in später beschriebener Weise drehangetrieben wird.

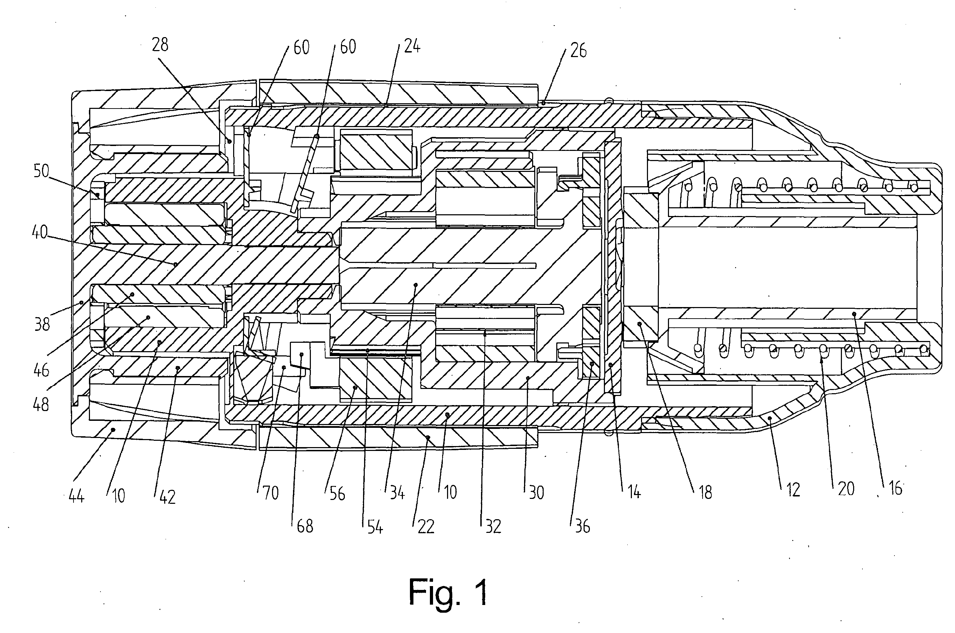

Das Gehäuse 10 wird von einer Außenhülse 22 umschlossen. Zwischen dem Außenumfang des Gehäuses 10 und der Außenhülse 22 werden axial verlaufende Luftkanäle 24 gebildet. Am vorderen Ende der Außenhülse 22 öffnen sich die Luftkanäle 24 mit einem Lufteintritt 26. Am hinteren Ende des Gehäuses 10 führen die Luftkanäle 24 um das hintere Ende des Gehäuses 10 herum durch einen Luftdurchtritt 28 in das Innere des Gehäuses. Im Inneren des Gehäuse 10 kann die Luft axial nach vorne strömen und an der Lagerstelle zwischen Drehbuchse 30 und Gehäuse 10 (Figur 1) hindurch in das Mundstück 12 gelangen. Nimmt der Benutzer das Mundstück 12 des Inhalators in den Mund und atmet ein, so saugt er Luft durch die Lufteintritte 26, die Luftkanäle 24, den Luftdurchtritt 28, das Gehäuse 10 und das Mundstück 12 an. Dabei nimmt die durch das Mundstück 12 eingeatmete Luft die durch die Frässcheibe 14 von der Wirkstofftablette 18 abgetragenen Wirkstoffpartikel mit, so dass diese in die Atemwege des Benutzers gelangen.

Die Frässcheibe 14 sitzt drehfest im axial vorderen Ende einer Drehbuchse 30, die koaxial drehbar in dem Gehäuse 10 gelagert ist. Der Drehantrieb der Drehbuchse 30 und damit der Frässcheibe 14 wird durch eine Antriebsfeder 32 bewirkt, die z. B. als schraubenförmig gewickelte Schenkelfeder oder Triebfeder aufgebildet ist. Die Antriebsfeder 32 sitzt koaxial in der Drehbuchse 30 und wird koaxial von einer Spannnabe 34 durchsetzt. Das eine Ende der Antriebsfeder 32 ist an der Drehbuchse 30 festgelegt, während das andere Ende der Antriebsfeder 32 an der Spannnabe 34 festgelegt ist. Der gegenseitige Drehwinkel zwischen der Drehbuchse 30 und der Spannnabe 34 wird durch einen Anschlagring 36 auf eine vorgegebene Anzahl von Umdrehungen begrenzt. Ein in die hintere axiale Stirnfläche des Inhalators eingesetztes Spannrad 38 greift mit einem axial mittigen Zapfen 40 drehmomentschlüssig in die Spannnabe 34. Das Spannrad ist einstückig mit einem hohlzylindrischen Außenring 42 ausgebildet, auf welchem über eine Friktionskupplung eine Spannkappe 44 sitzt. Die Spannkappe 44 schließt sich axial stufenlos an das hintere Ende der Außenhülse 22 an, während sich das Mundstück 12 axial stufenlos an das vordere Ende des Gehäuses 10 anschließt. Dadurch ergibt sich eine geschlossene zylindrische Außenform des Inhalators.

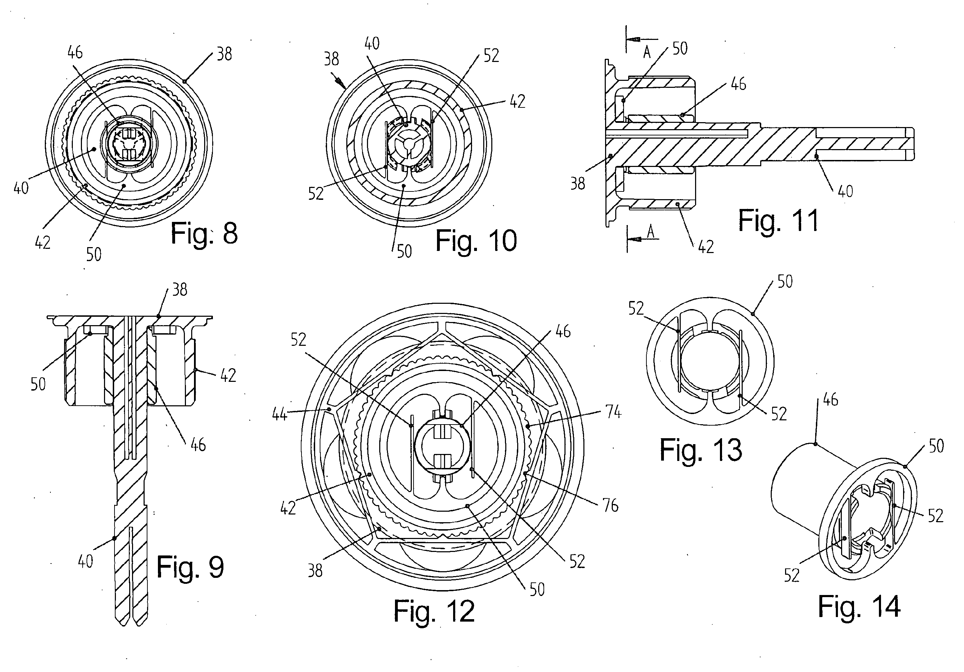

Auf dem Zapfen 40 des Spannrades 38 sitzt drehbar ein Schleppring 46. Der Schleppring 46 greift koaxial in das axial hintere Ende des Spannrades 38, welches seinerseits freidrehbar koaxial mit dem Außenring 42 in das Gehäuse 10 eingreift. Der Schleppring 46 ist dabei über einen vorgegebenen Winkelweg mit dem Spannrad 38 gekoppelt. Radial zwischen dem Schleppring 46 und dem hinteren Ende des Gehäuses 10 ist ein Freilauf 48 eingesetzt, der den Schleppring 46 mit dem Gehäuse 10 wirkungsmäßig verbindet. Zwischen der axial hinteren Stirnfläche des Gehäuses 10 und dem Spannrad 38 ist eine Pufferfederscheibe 50 eingesetzt, deren Form am deutlichsten aus den Figuren 13 und 14 ersichtlich ist. Die Pufferfederscheibe 50 weist einen Außenring auf, der drehfest an dem Spannrad 38 befestigt ist. An dem Außenring setzen diametral zwei Federarme 52 an, die nach Innen ragen und an diametralen Punkten von Außen an dem Schleppring 46 anliegen. Der Schleppring 46 weist in dem axialen Bereich, an welchem die Federarme 52 anliegen, Umfangsabflachungen auf.

Die Drehbuchse 30 ist mit einem Hemmregler ausgestattet, der ihre Drehbewegung auf eine vorgegebene Drehgeschwindigkeit regelt. Der Hemmregler weist ein auf dem Außenumfang der Drehbuchse 30 angeordnetes gezahntes Steigrad 54 auf, mit welchem ein freischwingender Anker 56 zusammen wirkt. Der Anker 56 ist als Ring ausgebildet, der das Steigrad 54 koaxial umschließt. Der Anker 56 weist in das Steigrad 54 eingreifende Ankerspitzen auf und ist im Gehäuse 10 mittels eines Lagerstiftes 58 schwenkbar gelagert.

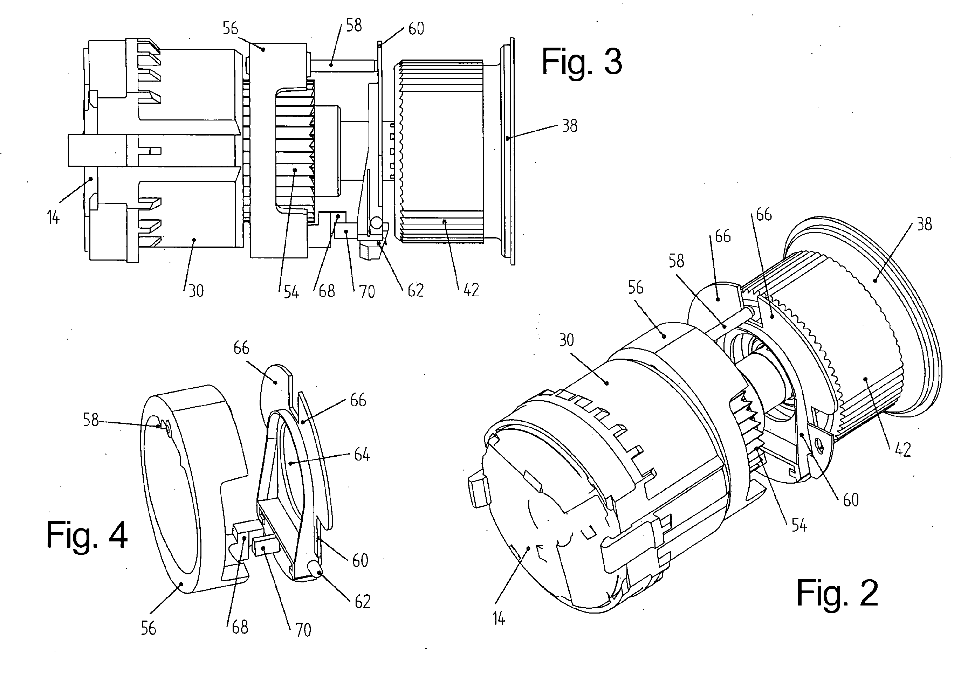

Axial hinter dem durch den Anker 56 und das Steigrad 54 gebildeten Hemmregler ist eine Auslöseeinrichtung angeordnet. Die Auslöseeinrichtung weist eine Luftklappe 60 auf, die um eine Schwenkachse 62 schwenkbar in dem Gehäuse 10 gelagert ist. Wie am deutlichsten in Figur 4 zu sehen ist, ist die Luftklappe 60 mit einem mittigen Durchbruch 64 ausgebildet, der von der Drehbuchse 30 durchsetzt wird. Die Schwenkachse 62 ist auf der einen Seite des Durchbruches 64 angeordnet und verläuft quer zur Mittelachse. Auf der der Schwenkachse 62 gegenüberliegenden Seite des Durchbruches ist die Luftklappe 60 mit großflächigen Klappenflügeln 66 ausgebildet. Die Klappenflügel 66 verschließen den Luftdurchtritt 28, durch welchen die angesaugte Luft von den Luftkanälen 24 in das Innere des Gehäuses 10 gelangt. Die Klappenflügel 66 lassen an ihrem Rand nur einen geringen Luftspalt des Luftdurchtrittes 28 frei. Der Luftdurchtritt 28 ist, wie in Figur 1 zu sehen ist, kreisbogenförmig in Bezug auf die Schwenkachse 62 als Mittelpunkt gekrümmt, so dass der Luftspalt zwischen den Klappenflügeln 66 und dem Luftdurchtritt 28 in jeder Winkelschwenkstellung der Luftklappe 60 im Wesentlichen gleich bleibt. Die Massenverteilung der Luftklappe 60 ist so ausgebildet, dass der Massenschwerpunkt mit der Schwenkachse 62 zusammenfällt. Der Lagerstift 58 des Ankers 56 ist diametral zu der Schwenkachse 62 der Luftklappe 60 angeordnet. Diametral zu dem Lagerstift 58 ist an dem Ring des Ankers 56 eine Fahne 68 angeformt, die radial verläuft und axial von dem Anker 56 nach hinten gegen die Luftklappe 60 hin absteht. Entsprechend weist die Luftklappe 60 im Bereich der Schwenkachse 62 eine Fahne 70 auf, die ebenfalls radial verläuft und axial nach vorne gegen den Anker 56 gerichtet von der Luftklappe 60 absteht. In der in Figur 1 mit 60 bezeichneten und in den Figuren 2 bis 4 dargestellten Sperrstellung der Luftklappe 60 ist die Luftklappe 60 um ihre Schwenkachse 62 so verschwenkt, dass sie senkrecht zur Mittelachse des Inhalators steht. In dieser Stellung wird die Luftklappe 60 durch eine schwache Rückholfeder 72 gehalten. In dieser Sperrstellung ist die Fahne 70 der Luftklappe 60 so radial nach Innen geschwenkt, dass sie in den Schwenkweg der Fahne 68 des Ankers 56 ragt. Bei der Schwingbewegung des Ankers 56 kommt dieser daher mit seiner Fahne 68 zur Anlage an der Fahne 70 der Luftklappe 60. Die Schwingbewegung des Ankers 56 wird dadurch behindert, so dass der Hemmregler eine Drehung der Drehbuchse 30 nicht mehr zulässt.

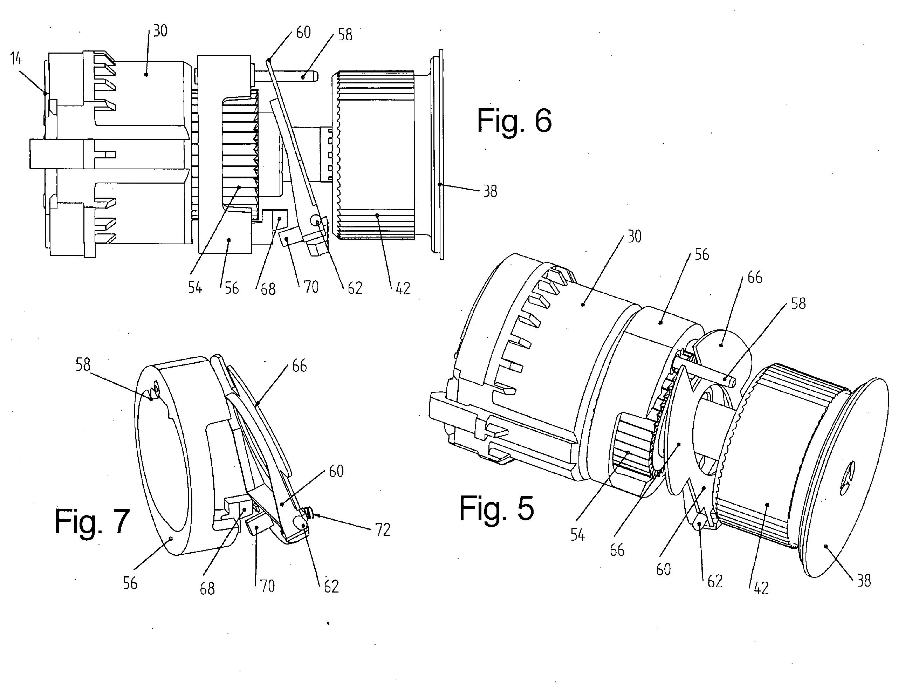

In der in Figur 1 mit 60' bezeichneten und in den Figuren 5 bis 7 dargestellten Auslösestellung der Luftklappe 60 ist diese so verschwenkt, dass ihre Fahne 70 radial nach außen aus dem Schwenkbereich der Fahne 68 des Ankers 56 geschwenkt ist. Der Anker 56 kann somit frei schwingen und die Drehbuchse 30 kann sich unter der Wirkung des Hemmreglers drehen.

### Die Funktionsweise des Inhalators ist folgende:

In der Ruhestellung wird die Luftklappe 60 durch die Rückholfeder 72 in die Sperrstellung geschwenkt, in welcher die Luftklappe 60 eine Schwingbewegung des Ankers 56 verhindert. Das Gehäuse 10 mit der Außenhülse 22 ist dadurch über die Luftklappe 60, den Anker 56 und das Steigrad 54 drehfest mit der Drehbuchse 30 gekuppelt. Nun kann der Federantrieb gespannt werden. Hierzu wird das Spannrad 38 mittels der Spannkappe 44 gegenüber dem mittels der Außenhülse 22 festgehaltenen Gehäuse 10 verdreht. Das Spannrad 38 nimmt die Spannnabe 34 mit und verdreht diese gegenüber der gehäusefest gehaltenen Drehbuchse 30, so dass die Antriebsfeder 32 gespannt wird. Der Freilauf 48 lässt diese Drehung in Spannrichtung zu, verhindert jedoch ein Zurückdrehen des Schleppringes 46. Das Spannrad 38 und die Spannnabe 34 können unter der Wirkung der gespannten Antriebsfeder 32 um einen vorgegebenen Winkelweg zurückdrehen. Die Antriebsfeder 32 kann soweit gespannt werden, bis die Spannnabe 34 an dem Anschlagring 36 zum Anschlag kommt. Bei einem weiteren Überdrehen der Spannkappe 44 kommt eine Überdrehsicherung mittels Friktionskupplung zur Wirkung. Diese Friktionskupplung setzt sich aus einer Verzahnung 74 auf dem Außenring 42 und Rastmembranen 76 zusammen

Wenn beim Spannen der Antriebsfeder 32 die Spannnabe 34 gegen den Anschlagring 36 anschlägt, können sich die unter Torsion belastete Teile elastisch verformen und dabei eine elastische Verformungsenergie speichern.

Beim Entlasten des Spannrades 38 über die Spannkappe 44 bewirkt die elastische Verformungsenergie und das Rückstellmoment der Triebfeder ein Zurückdrehen des Spannrades 38 um das Verdrehspiel zwischen Schleppring 46 und Spannrad. Dabei verformen sich die Federarme 52 der Pufferscheibe 50, wobei der Schleppring 46 durch den Freilauf 48 verdrehgesichert ist. Durch diese Rückdrehung wird die gespeicherte Verformungsenergie abgebaut.

Wird der Inhalator benutzt, so steckt der Benutzer das Mundstück 12 in den Mund und atmet ein. Dadurch wird Luft über die Lufteintritte 26, die Luftkanäle 24, den Luftdurchtritt 28 und das Mundstück 12 angesaugt. Die dadurch in dem Luftdurchtritt 28 erzeugte Luftströmung nimmt die Klappenflügel 66 mit und verschwenkt dadurch die Luftklappe 60 in die Auslösestellung. Dadurch wird die Fahne 70 der Luftklappe 60 aus dem Schwenkweg der Fahne 68 des Ankers 56 geschwenkt und der Anker 56 kann frei schwingen. Dadurch ist die drehschlüssige Kupplung zwischen dem Gehäuse 10 und der Drehbuchse 30 aufgehoben. Die Drehbuchse 30 kann sich unter der Wirkung der Antriebsfeder 32 mit der durch den Hemmregler vorgegebenen Drehgeschwindigkeit drehen. Dadurch dreht sich die Frässcheibe 14 gegenüber der über das Mundstück 12 Gehäuse festgehaltenen Wirkstofftablette 18 und trägt eine definierte Menge an Wirkstoffpartikeln ab, die von dem Benutzer über das Mundstück 12 eingeatmet werden. Die Drehbuchse 30 mit der Frässcheibe 14 kann dabei einen Drehweg zurücklegen, dessen Länge durch den Anschlagring 36 festgelegt ist.

### Bezugszeichenliste

- 10: Gehäuse
- 12: Mundstück
- 14: Frässcheibe
- 16: Trägerhülse
- 18: Wirkstofftablette
- 20: Schraubendruckfeder
- 22: Außenhülse
- 24: Luftkanäle
- 26: Lufteintritt
- 28: Luftdurchtritt
- 30: Drehbuchse
- 32: Antriebsfeder
- 34: Spannnabe
- 36: Anschlagring
- 38: Spannrad
- 40: Zapfen
- 42: Außenring
- 44: Spannkappe
- 46: Schleppring
- 48: Freilauf
- 50: Pufferfederscheibe
- 52: Federarme
- 54: Steigrad
- 56: Anker
- 58: Lagerstift
- 60: Luftklappe
- 62: Schwenkachse
- 64: Durchbruch
- 66: Klappenflügel
- 68: Fahne von 56
- 70: Fahne von 60
- 72: Rückholfeder
- 74: Verzahnung
- 76: Rastmembran

## Patentansprüche

1. Inhalator mit einer drehantreibbaren Abtrageinrichtung, mit einem Drehantrieb für die Abtrageinrichtung, mit einem Wirkstoffträger, der an der Abtrageinrichtung zur Anlage gebracht wird, um Wirkstoffpartikel abzutragen, mit einem Mundstück, mit wenigstens einem Luftkanal, durch welchen Luft zu dem Mundstück angesaugt wird und die abgetragenen Wirkstoffpartikel mitnimmt, und mit einer Auslöseeinrichtung für den Drehantrieb,
**dadurch gekennzeichnet, dass** die Auslöseeinrichtüng (60) in dem wenigstens einen Luftkanal (28) angeordnet ist und durch den durch den Luftkanal (28) angesaugten Luftstrom von einer den Drehantrieb sperrenden Stellung in eine dem Drehantrieb auslösende Stellung bewegbar ist.

2. Inhalator nach Anspruch 1,
**dadurch gekennzeichnet, dass** die Auslöseeinrichtung eine Luftklappe (60) aufweist, die in den Luftkanal (28) hineinragt.

3. Inhalator nach Anspruch 2,
**dadurch gekennzeichnet, dass** die Luftklappe (60) um eine Schwenkachse (62) schwenkbar ist und dass der Masseschwerpunkt der Luftklappe (60) im Wesentlichen in der Schwenkachse (62) liegt.

4. Inhalator nach einem der vorhergehenden Ansprüche,
**dadurch gekennzeichnet, dass** der Drehantrieb ein Federantrieb ist.

5. Inhalator nach Anspruch 4,
**dadurch gekennzeichnet, dass** die Auslöseeinrichtung (60) in der Sperrstellung mechanisch in den Drehantrieb eingreift und dessen Drehbewegung blockiert.

6. Inhalator nach Anspruch 5,
**dadurch gekennzeichnet, dass** der Federantrieb einen Hemmregler (54, 56) aufweist.

7. Inhalator nach Anspruch 6,
**dadurch gekennzeichnet, dass** die Auslöseeinrichtung (60) in der Sperrstellung die Schwingbewegung des Hemmreglers (54, 56) blockiert.

8. Inhalator nach den Ansprüchen 3 und 7,
**dadurch gekennzeichnet, dass** die schwenkbare Luftklappe (60) in der Sperrstellung mit einer Fahne (60) axial in den Schwenkweg einer Fahne (68) des Ankers (56) des Hemmreglers eingreift.

9. Inhalator nach Anspruch 4,
**dadurch gekennzeichnet, dass** der Federantrieb eine mittels einer drehbaren Spanneinrichtung (34, 38, 40) über einen Freilauf (48) aufziehbare Antriebsfeder (32) aufweist.

10. Inhalator nach Anspruch 9,
**dadurch gekennzeichnet, dass** der Federantrieb auf einen im Wesentlichen konstanten Bereich der Federcharakteristik der Antriebsfeder (32) durch Anschläge (36) begrenzt ist.

11. Inhalator nach Anspruch 10,
**dadurch gekennzeichnet, dass** eine unter Friktion verdrehbare Spannkappe (44) ein Zerstören des Anschlags (36) beim Spannen der Antriebsfeder (32) verhindert.

12. Inhalator nach Anspruch 10 oder 11,
**dadurch gekennzeichnet, dass** elastische Verformungsspannungen bei dem den Federaufzug begrenzenden Anschlag durch einen Schleppanschlag mit Pufferfeder (50) abgebaut werden.

13. Inhalator nach einem der Ansprüche 1 bis 3,
**dadurch gekennzeichnet, dass** die Auslöseeinrichtung einen elektrischen Kontakt betätigt und damit einen batteriegespeisten elektromotorischen Drehantrieb auslöst.
